(19) Europäisches Patentamt
European Patent Office
Office européen des brevets

(11) **EP 3 229 859 B1**

(12) **EUROPÄISCHE PATENTSCHRIFT**

(45) Veröffentlichungstag und Bekanntmachung des Hinweises auf die Patenterteilung:
**06.02.2019 Patentblatt 2019/06**

(51) Int Cl.:
***A61M 1/36*** *(2006.01)*      ***A61M 1/16*** *(2006.01)*
***A61M 1/34*** *(2006.01)*

(21) Anmeldenummer: **15804319.0**

(22) Anmeldetag: **02.12.2015**

(86) Internationale Anmeldenummer:
**PCT/EP2015/002429**

(87) Internationale Veröffentlichungsnummer:
**WO 2016/091366 (16.06.2016 Gazette 2016/24)**

(54) **DIALYSEMASCHINE ZUR ÜBERWACHUNG ZEITLICHE VERÄNDERUNGEN DES HÄMATOKRITS UND/ODER HÄMOGLOBINSWERTES.**

DIALYSIS MACHINE FOR MONITORING THE TIME CHANGES OF THE HEMATOCRIT AND/OR HEMOGLOBIN VALUE

MACHINE DE DIALYSE POUR LA SURVEILLANCE DE VARIATIONS TEMPORELLES DU HÉMATOCRITE ET/OU DES VALEURS D'HÉMOGLOBINE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **08.12.2014 DE 102014018072**

(43) Veröffentlichungstag der Anmeldung:
**18.10.2017 Patentblatt 2017/42**

(73) Patentinhaber: **Fresenius Medical Care Deutschland GmbH**
**61352 Bad Homburg (DE)**

(72) Erfinder:
• **WIENEKE, Paul**
**48149 Hamburg (DE)**

• **WOJKE, Ralf**
**61348 Bad Homburg (DE)**

(74) Vertreter: **Herrmann, Uwe**
**Lorenz Seidler Gossel**
**Rechtsanwälte Patentanwälte**
**Partnerschaft mbB**
**Widenmayerstraße 23**
**80538 München (DE)**

(56) Entgegenhaltungen:
WO-A1-88/02864      WO-A1-03/074109
WO-A1-2010/040927      WO-A1-2014/044365
WO-A1-2014/083448      WO-A1-2014/095073

**Beschreibung**

Die vorliegende Erfindung betrifft eine Dialysemaschine mit einem extrakorporalen Kreislauf, in dem sich ein Dialysator befindet, der eine von Blut durchströmte blutseitige Kammer aufweist, sowie mit einem in Strömungsrichtung des Blutes stromaufwärts der blutseitigen Kammer befindlichen ersten Drucksensor zur Ermittlung eines ersten Druckwertes und einem in Strömungsrichtung des Blutes stromabwärts der Kammer befindlichen zweiten Drucksensor zur Ermittlung eines zweiten Druckwertes.

Derartige Dialysemaschinen sind in unterschiedlichen Ausführungen aus dem Stand der Technik bekannt. Das Blut des Patienten wird mittels einer Blutpumpe durch den extrakorporalen Kreislauf gefördert. In dem Dialysator erfolgt eine Reinigung bzw. ein Wasserentzug über die Dialysatormembran, die üblicherweise aus einer Vielzahl von Kapillarwandungen besteht. Das auf diese Weise gereinigte Blut gelangt zurück zum Patienten. Während bei der Hämodialyse ein überwiegend diffusiver Stofftransport über die Membran erfolgt, kommt bei der Hämodiafiltration ein durch ein Druckgefälle über die Membran bedingter konvektiver Stofftransport hinzu. Letzterer ist bei der Hämofiltration die wesentliche Größe, die die Blutreinigung bzw. Entwässerung bewirkt.

Nach dem Gesetz von Hagen-Poiseuille besteht bei laminarer Strömung ein linearer Zusammenhang zwischen den transportierten Flüssigkeitsvolumen V/t und der Druckdifferenz $\Delta$p entlang der Membran, d.h. der Druckdifferenz zwischen dem Anfang und dem Ende der Kapillare der Länge L, nach folgender Gleichung:

$$(1) \qquad V/t = (\pi\ r^4\ \Delta p)\ /\ (8\ \eta\ L)$$

V/t (auch als Q bezeichnet) ist der Blutfluss durch die Kapillare, r ist deren innerer Radius und $\eta$ die dynamische Viskosität des Blutes.

Dialysatoren weisen üblicherweise eine große Anzahl n von Kapillaren auf (n > 10.000), die einen kleinen Radius (80 $\mu$m < r < 110 $\mu$m) aufweisen, damit eine möglichst große Membranoberfläche und somit Austauschoberfläche erhalten wird. Die Membranoberfläche A ergibt sich dann insgesamt zu A = 2 $\pi$ r L n.

Dialysatoren mit gleicher Membranoberfläche A können bei gleichem $\Delta$p ein unterschiedliches Flüssigkeitsvolumen transportieren bzw. bei gleichem Q einen unterschiedlichen Druckabfall bewirken, da die Dialysatoren sich in den Parametern r, L und n unterscheiden können. Bei einer Newtonschen Flüssigkeit hat jeder einzelne Parameter (n, L, r) schon im Idealfall unterschiedliche Auswirkungen auf den Flüssigkeitstransport, wie sich dies aus Gleichung (1) ergibt. Der gesamte Flüssigkeitstransport durch den Dialysator ist proportional zu n.

Ein weiterer Faktor, der auf den Flüssigkeitstransport einen Einfluss haben kann, ist die Anzahl und Geometrie der Poren in der Membranoberfläche bzw. die Rauigkeit $\varepsilon$ der Membranoberfläche. Berücksichtigt man diese in der oben genannten Gleichung (1), könnte diese in Form der modifizierten Gleichung (2)

$$(2) \qquad V/t = (\pi\ r^4\ \Delta p)\ /\ (8\ \eta\ L\ \varepsilon)$$

erfasst werden, aus der hervorgeht, dass die Durchflussrate mit steigender Rauigkeit abnimmt.

Diese Rauigkeit bzw. die weiteren charakteristischen Eigenschafen kennzeichnen den Dialysator und können durch eine Markierung bzw. Messung im Werk oder auch bei Beginn einer Dialysebehandlung ermittelt werden. Eine solche Ermittlung kann durch Druckmessungen stromaufwärts und stromabwärts direkt am Dialysator sowohl auf der Blut- als auch auf der Dialysatseite erfolgen.

Bei einer laminaren Durchströmung der Kapillare ist der Druckverlust über die Länge der Kapillare proportional zur mittleren Geschwindigkeit des Blutes in der Kapillare.

Beim Übergang vom laminaren zum turbulenten Fluss ändert sich das parabelförmige Profil der Strömung mehr zu einer weitgehend gleichmäßigen Geschwindigkeit über die Querschnittsfläche der Kapillare. Die turbulente Schwankungsbewegung hat einen erhöhten Druckverlust $\Delta$p über die Länge der Kapillare und eine erhöhte Wandschubspannung zur Folge. In der turbulenten Strömung ist der Druckverlust über die Kapillarlänge proportional zum Quadrat der mittleren Geschwindigkeit.

Aufgrund des Flüssigkeitsentzuges über die Membran ändert sich die dynamische Viskosität des Blutes zunehmend.

Bei massivem Flüssigkeitsentzug über die Membran stellt sich in der Kapillarmitte eine massive lokale Erhöhung der Zelldichte und des lokalen Hämatokrits ein. Die Zellen nehmen an der Membran aufgrund des Flüssigkeitsentzugs eine immer dichtere Anordnung ein, die zu einer Versteifung des Blutes führt. Das Blut wird sich mit einer über den Querschnitt weitgehend gleichförmigen Geschwindigkeit durch die Hohlfaser bewegen.

Bei modernen Kapillardialysatoren ist die Näherung einer laminaren Rohrströmung nicht mehr generell zutreffend. Die Kapillaren werden häufig onduliert, die dadurch bedingten Kurven stören den laminaren Fluss. Es existieren kleinere Membranoberflächen und reduzierte Kapillarradien, die sich ebenfalls negativ auf die laminare Strömung auswirken.

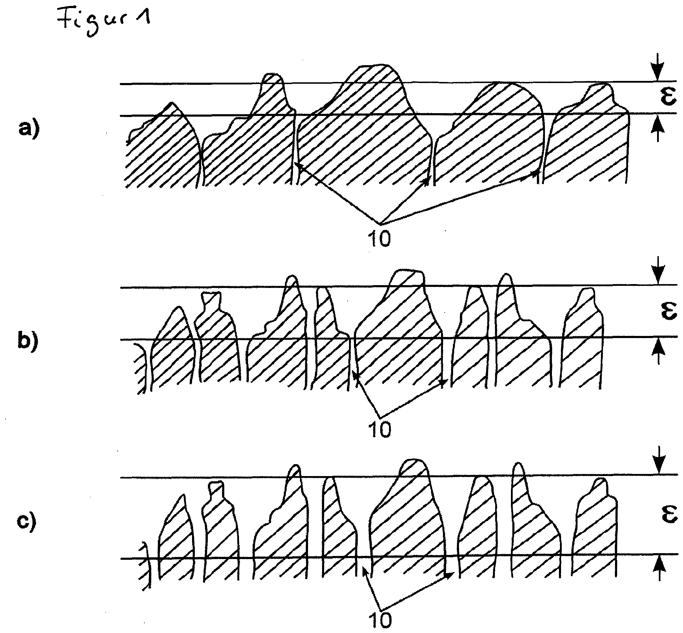

Bei modernen Kapillaren befinden sich mehr Poren in der Membran, wodurch die Flüssigkeitsdurchlässigkeit durch die Membran sowie auch die Rauigkeit $\varepsilon$ der Membran erhöht, wie dies aus Kapillarwände im Schnitt zeigenden Figur 1 hervorgeht, die die Rauigkeiten für eine Low-Flux Membran a), für eine High-Flux Membran b) und für die High-Cut off Membran c) zeigt. Die Kapillarinnenseite befindet sich jeweils oben. Die Poren sind mit dem Bezugszeichen 10 gekennzeichnet.

Bei modernen Dialyseverfahren, wie z.B. dem Hämodiafiltrationsverfahren, kann es zu einer massiven Erhöhung des Hämatokrits bis zu 70 bis 80 % kommen, der zumindest lokal zu der oben genannten Versteifung des Blutes führt.

Ausgehend von dem Eingangsbereich der Kapillare mit einem physiologischen Hämatokrit und laminarem Blutfluss, ergibt sich in einem Übergangsbereich ein durch Ultrafiltration unphysiologisch erhöhter Hämatokrit und eine nicht mehr homogene Strömung. Es bilden sich Zellaggregate, das parabelförmige Geschwindigkeitsprofil flacht ab. Im Endbereich ist der Hämatokrit im gesamten Blutvolumen extrem unphysiologisch, das Blut versteift und das Geschwindigkeitsprofil wird propfenförmig. Der lokale Druckabfall steigt stark an und die Grenzschicht zwischen dem Blut und der Membran löst sich auf. Es kommt zu erheblichen Interaktionen zwischen Zellen und der Membran und ggf. zu Zellschädigungen.

Innerhalb der Hohlfaser treffen durch Flüssigkeitsentzug und Eindickung die Zellen aufeinander, d.h. die Zellen können sich nicht mehr frei bewegen, sondern wirken aufeinander ein. Bei größeren Kapillardurchmessern kann dies zu Zelldeformationen führen, bei kleineren Kapillardurchmessern kann eine Umgruppierung und Deformation der Zellen stattfinden.

Im Nahbereich der Kapillarwand werden Blutzellen mit mittlerer Blutgeschwindigkeit an der Kapillarwand entlang geführt und erfahren unter Umständen turbulente Schwankungsbewegungen, erhöhte Wandschubspannungen und Scherkräfte sowie durch den Kontakt mit der rauen Kapillarwand deformierende und abrasive Effekte.

Mit der Ultrafiltration von flüssigen Bestandteilen des Blutes über die Kapillarmembran werden die zellulären Bestandteile eingedickt und auf die Membran aufgeschoben. Je nach mechanischer Konsistenz und dem Aufbau der Kapillarmembran, insbesondere im Bereich der Poren sind auch deformierende und abrasive Effekte an den zellulären Membranen denkbar.

Die Wechselwirkung von rasch fließendem Blut und der Kapillarmembran kann unter Umständen auch die Kapillarmembran selbst beeinflussen. So ist es beispielsweise denkbar, dass die Poren verändert werden, was seinerseits Einfluss auf das Blut bzw. auf dessen Bestandteile haben kann.

Zusammenfassend lässt sich feststellen, dass die oben genannten Effekte unter Umständen zu einer Beeinträchtigung der Integrität der Blutzellen führen können: diese Beeinträchtigung kann je nach Zelltyp zu einer Deformation, zu einer Reduktion der Lebensdauer der Zellen, bis hin zu einer Zerstörung der Zellen (Hämolyse) oder auch zu Ausschüttungen von zellulären Bestandteilen, ggf. mit systemischen Folgen führen (Zytokine).

Wird ausgehend von niedrigen Werten die Blutflussrate erhöht, kann der Bereich des laminaren Blutflusses verlassen werden, d.h. das im wesentlichen parabolische Geschwindigkeitsprofil wird verlassen und es wird ein mittleres über die Querschnittsfläche gleichmäßiges Geschwindigkeitsprofil erzeugt. Der Umschlagpunkt ist von der Viskosität und von der Geometrie der Hohlfaser abhängig. Dieser Übergang, der mit einer Versteifung des Blutes einhergeht, ist fließend.

Die Ultrafiltration führt zu einem Flüssigkeitsentzug über die Membran, d.h. über die Hohlfaser bzw. Kapillaren ist prinzipiell über die gesamte Kapillarlänge wirksam. Durch den Flüssigkeitsentzug wird die Viskosität des Blutes verändert. Wird die Ultrafiltration stark erhöht, kann sich der Charakter des Blutes von newtonsch zu nicht-newtonsch verändern. Das Fließverhalten des Blutes ändert sich dadurch dahingehend, dass das Geschwindigkeitsprofil in der Kapillarmitte abgeflacht wird, d.h. das parabolische Profil mit einer größten Geschwindigkeit in der Kapillarmitte und einer geringsten Geschwindigkeit an der Kapillarwand verflacht sich. Die Strömung ist jedoch nicht turbulent, sondern versteift.

Zusammenfassend ergibt sich, dass eine hohe Blutflussrate und eine hohe Ultrafiltrationsrate die Wahrscheinlichkeit für das Auftreten von Zellschädigungen des Blutes erhöhen kann.

Die Fragilität der Blutzellen ist in erster Näherung von genetischen Faktoren, der Frequenz und der Intensität der Nierenersatztherapie, der Nierenrestfunktion sowie anderer Systemerkrankungen abhängig und von Patient zu Patient unterschiedlich. Der Übergang vom newtonschen zu nicht-newtonschen Verhalten hängt u.a. vom Anteil der zellulären Bestandteile (Hämatokrit) ab.

Der vorliegenden Erfindung liegt die Aufgabe zugrunde, eine Dialysemaschine der eingangs genannten Art dahingehend weiterzubilden, dass die Wahrscheinlichkeit für das Auftreten derartiger Zellschädigungen durch die Eindickung des Blutes verringert wird.

Diese Aufgabe wird durch eine Dialysemaschine mit den Merkmalen des Anspruchs 1 gelöst.

Danach ist vorgesehen, dass die Dialysemaschine erste Mittel aufweist, die die Druckdifferenz zwischen dem zweiten und dem ersten Druckwert ermitteln, dass die Dialysemaschine zweite Mittel aufweist, die die dynamischen Viskosität des Blutes basierend auf der ermittelten Druckdifferenz, der Blutflussrate durch die blutseitige Kammer und einer oder mehrerer charakteristischer Eigenschaften des Dialysators ermitteln, dass die Dialysemaschine dritte Mittel aufweist, die den Hämatokrit oder den Hämoglobinwert des Blutes im Dialysator basierend auf der ermittelten Viskosität ermitteln und dass die Dialysemaschine eine Steuer- oder Regelungseinheit aufweist, die derart ausgebildet ist, dass diese die Blutflussrate und/oder die Dilutionsrate und/oder die Ultrafiltrationsrate derart einstellt, dass die zeitliche Veränderung des Hämatokrits und/oder des Hämoglobinwertes einen Grenzwert nicht übersteigt oder einem Sollwertbereich liegt.

Gemäß der vorliegenden Erfindung wird somit die blutseitige Druckdifferenz (vorzugsweise unmittelbar) vor und nach dem Dialysator gemessen und darauf basierend die dynamische Viskosität des Blutes ermittelt. Gemäß Gleichung (2) sind weitere Parameter, die in die Bestimmung der Viskosität einfließen, der Blutfluss (V/t) sowie Eigenschaften des Dialysators, insbesondere dessen laminarer Strömungswiderstand, der z.B. im Rahmen der Dialysatorproduktion festgestellt werden kann.

Aus der ermittelten Viskosität kann dann der Hämatokrit und/oder der Hämoglobinwert bestimmt werden.

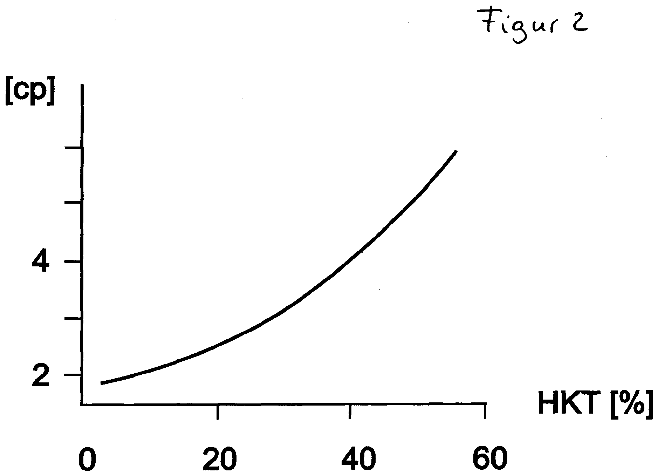

Figur 2 zeigt einen exemplarischen Zusammenhang zwischen dem Hämatokrit HKT und der dynamischen Viskosität η.

Die in Figur 2 wiedergegebene Beziehung gilt für einen idealisierten Patienten und kann mit der damit verbundenen Unschärfe für alle Patienten verwendet werden. Vorteilhaft ist es jedoch, eine patientenindividuelle Beziehung heranzuziehen, um so ein möglichst genaues Ergebnis für den Hämatokrit und/oder der Hämoglobinwert zu erhalten.

Die Steuer- oder Regelungseinheit der Dialysemaschine vergleicht den zeitlichen Anstieg des Hämatokrites und/oder der Hämoglobinwertes und vergleicht diesen mit einem oberen Grenzwert bzw. prüft, ob dieser in einem Sollwertbereich liegt.

Ist dies der Fall, bedarf es keines Eingriffes durch die Dialysemaschine, da ein gewisser Anstieg während der Behandlung normal ist.

Kommt es jedoch zu einer Grenzwertüberschreitung oder zu einem Verlassen des Sollwertbereiches, ändert die Dialysemaschine veranlasst durch die Steuer- oder Regelungseinheit die Blutflussrate über eine Änderung der Geschwindigkeit der Blutpumpe und/oder die Substitutionsrate über die Änderung der Geschwindigkeit einer Substitutionspumpe, mit der ein Substitutionsfluid dem extrakorporalen Kreislauf zugeführt wird. Alternativ oder zusätzlich wird die Ultrafiltrationsrate geändert.

Vorzugsweise ist jedoch vorgesehen, dass die Ultrafiltrationsrate nicht zur Einstellung der zeitlichen Veränderung des Hämatokrits und/oder des Hämoglobinwertes herangezogen wird, da diese zur Erreichung des gewünschten Trockengewichts des Patienten einen bestimmten Wert oder ein bestimmtes Profil annehmen soll.

Der Grenzwert oder der Sollwertbereich kann fest vorgegeben sein oder experimentell ermittelt werden, wie z.B. durch Zunahme der Zelldebris oder freiem Hämoglobin bei simulierter Dialysebehandlung im Blutlabor.

Die Eigenschaften des Dialysators, die in die Ermittlung der Viskosität einfließen, können den sich bei laminarer Strömung ergebenden Strömungswiderstand des Dialysators umfassen. Dieser kann beispielsweise nach der Herstellung des Dialysators im Werk oder vor der Behandlung mittels einer Spülflüssigkeit oder auch am Anfang der Behandlung mittels des Blutes selbst ermittelt werden.

Denkbar ist es weiterhin, dass die blutseitige Kammer des Dialysators aus einer Mehrzahl von Kapillaren besteht und dass die Eigenschaften des Dialysators die Länge der Kapillaren und/oder deren Innenabmessungen und/oder deren Anzahl und/oder deren Form und/oder deren Wandstruktur und/oder die Rauigkeit der Kapillarwandungen umfassen.

Die Eigenschaften des Dialysators, insbesondere dessen Strömungswiderstand, lassen sich durch Messung des Druckabfalls über die Kapillarlänge $\Delta p$ bei bestimmten Flüssen und Medien aus Gleichung (1) oder (2) ermitteln.

Sind die Eigenschaften des Dialysators bekannt, kann bei bekanntem bzw. gemessenem Blutfluss (V/t bzw. Q) durch die Messung des Druckverlustes über die Kapillarlänge die dynamische Viskosität η des Blutes bestimmt werden.

In einer weiteren Ausgestaltung der Erfindung ist vorgesehen, dass die dritten Mittel einen Speicher aufweisen, in dem ein Zusammenhang zwischen Hämatokrit oder Hämoglobinwert des Blutes und der Viskosität abgelegt ist und dass die dritten Mittel derart ausgeführt sind, dass der Hämatokrit oder der Hämoglobinwert des Blutes basierend auf diesem Zusammenhang bestimmt wird. Wie in Figur 2 dargestellt, besteht ein nicht linearer Zusammenhang zwischen beiden Größen.

Denkbar ist es, dass der Zusammenhang fest vorgegeben ist. In diesem Fall wird patientenunabhängig eine feste Beziehung zwischen Hämatokrit und Viskosität verwendet.

Wie bei konkreten Patienten der Blutströmungswiderstand z.B. bei Beginn der Behandlung bestimmt, so lässt sich durch einen Vergleich mit dem labormäßig bekannten Hämatokrit bzw. Hämoglobin ein Korrekturfaktor errechnen, der patientenindividuell die Genauigkeit dieser Blutviskositäts/Hämatokrit-Kurven und somit die Genauigkeit des ermittelten Hämatokrits und/oder des Hämoglobinwertes verbessert.

Die dritten Mittel können derart ausgebildet sein, dass der Hämatokrit oder Hämoglobinwert durch Interpolation oder durch Extrapolation bestimmt wird. Auch andere Berechnungsarten sind denkbar und von der Erfindung mit umfasst.

Ist der Dialysator hinreichend genau bestimmt, d.h. sind dessen charakteristische Eigenschaften ermittelt, kann aus den gemessenen Druckwerten am Dialysator auf die Viskosität und von dieser auf den Hämatokrit und/oder den Hämoglobinwert geschlossen werden. Wird zudem noch eine patientenindividuelle Patientenkalibrierung durchgeführt, lassen sich Einflüsse des Blutes und individuelle möglicherweise pathologische Einflüsse auf die Blutviskosität abfangen bzw. berücksichtigen.

Ist der Dialysator hinreichend genau bestimmt, d.h. sind dessen charakteristische Eigenschaften ermittelt, kann aus einem Anstieg des gemessenen Druckabfalls über die Dialysatorlänge auf die Viskosität und von dieser auf den Hämatokrit und/oder der Hämoglobinwert bzw. deren zeitliche Änderung geschlossen werden.

Vorzugsweise sind die ersten bis dritten Mittel ohnehin vorhandene Bestandteile einer Dialysemaschine sind, so dass kein spezielles, zusätzliches Disposable etc. notwendig ist, wodurch Kosten eingespart werden können.

Denkbar ist es, dass die charakteristischen Eigenschaften des Dialysators durch Messung mit Blut oder durch Messung mit einer anderen Flüssigkeit, insbesondere durch Messung mit einer Spülflüssigkeit erhalten werden. So kann eine "Kalibrierung", d.h. die Ermittlung des blutseitigen Strömungswiderstandes während oder nach dem Primen durchgeführt werden. Auch ist es möglich, eine derartige Bestimmung werkseitig durchzuführen. Ist die Viskosität der Flüssigkeit bekannt oder lässt sich diese durch mehrere Messungen aus dem Gleichungssystem eliminieren und wird der Druckverlust über die Länge der Kapillaren gemessen, lassen sich daraus die charakteristischen Eigenschaften und insbesondere der Strömungswiderstand des Dialysators ermitteln.

Diese Kalibrierung kann z.B. auch durch Mehrpunkt-Messungen bei verschiedenen Fluss- und Ultrafiltrationsraten durchgeführt werden.

Die Dialysemaschine kann eine von Dialyselösung durchströmte dialysatseitige Kammer sowie einen in Strömungsrichtung der Dialyselösung stromaufwärts dieser Kammer befindlichen dritten Drucksensor zur Ermittlung eines dritten Druckwertes und einen in Strömungsrichtung der Dialyselösung stromabwärts dieser Kammer befindlichen vierten Drucksensor zur Ermittlung eines vierten Druckwertes aufweisen.

Bei der Dialysemaschine kann es sich um eine Hämodialysemaschine, um ein Hämofiltrationsgerät oder um ein Hämodiafiltrationsgerät handeln. Dementsprechend kann unter dem Begriff "Dialysator" ein Hämodialysator oder auch ein Hämofilter verstanden werden, der bei der Hämodiafiltration oder bei der Hämofiltration zum Einsatz kommt.

Die Dialysemaschine weist vorzugsweise eine Predilutionsleitung auf, die in Strömungsrichtung des Dialysators stromaufwärts des Dialysators in den extrakorporalen Kreislauf mündet. Bei der Dilutionsrate handelt es sich in diesem Fall vorzugsweise um die Infusionsrate des durch die Predilutionsleitung zugeführten Substitutionsfluids. Auch eine Postdilution ist möglich und von der Erfindung umfasst.

Weitere Einzelheiten und Vorteile der Erfindung werden anhand eines in der Zeichnung dargestellten Ausführungsbeispiels näher erläutert. Es zeigen:

Figur 1:     unterschiedliche schematische Längsschnittansichten durch verschiedene Membrantypen und

Figur 2:     eine schematische Darstellung der Abhängigkeit der Blutviskosität vom Hämatokrit.

Vor Beginn der Dialysebehandlung wird der blutseitige Strömungswiderstand über die im Hämodialysegerät vorhandenen Drucksensoren gemessen, d.h. die "Kalibrierung" erfolgt nicht im Werk. Diese Messung kann automatisch während oder auch nach dem Primen der Dialysemaschine durchgeführt werden.

Während des Primens erfolgt die Messung z.B. mittels einer Spüllösung, wie einer physiologischen Kochsalzlösung. Nach dem Primen erfolgt die Messung mittels des Blutes des Patienten.

Im ersten Fall existiert der Vorteil, dass der Patient nicht anwesend sein muss, jedoch der Nachteil, dass nur der Strömungswiderstand mit einer Kochsalzlösung ermittelt wird, was für den Blutwiderstand bei der Durchströmung mit Blut nur eine Näherung darstellt.

Alternativ zu dieser Vorgehensweise kann die "Kalibrierung" in der Produktionsstätte des Dialysators erfolgen und eine Übermittlung des Ergebnisses an die Dialysemaschine erfolgen.

In den blutseitigen Widerstand gehen u.a. die Geometrie des Dialysators ein, wie z.B. die Anzahl der Kapillaren, deren Länge, der innere Kapillarradius, der Aufbau der Membran (wie z.B. die Anzahl, die Größe und die Geometrie der Poren) sowie ggf. die Eigenschaften der verwendeten Materialien (wie z.B. die Elastizität, Härte, Compliance).

Denkbar ist es, dass nach der Produktion einer Charge von Dialysatoren der erste und der letzte Dialysator vermessen werden und die Dialysatoren der gesamten Charge mit dem Wert oder Mittelwert des ermittelten Strömungswiderstandes versehen werden (z.B. auf einem RFID-Chip, Barcode oder einem anderen geeigneten Datenspeicher). Dieser Wert kann zur Behandlung, vorzugsweise vor der Behandlung seitens der Dialysemaschine eingelesen werden.

Ist der Dialysator auf diese Weise kalibriert, d.h. sind dessen Eigenschaften und insbesondere dessen Strömungswiderstand bekannt, kann über die Messung des Druckverlustes über die Länge des Dialysators die dynamische Viskosität ermittelt werden.

Basierend darauf lässt sich aus der Viskosität der Hämatokrit und/oder der Hämoglobinwert ermitteln. Um die Genauigkeit dieser Ermittlung zu verbessern, kann vorgesehen sein, dass patientenindividuell zu Beginn der Behandlung der Blutströmungswiderstand gemessen wird und durch Vergleich mit dem labormäßig bekannten Hämatokrit und/oder der Hämoglobinwert ein Korrekturfaktor errechnet wird, der patientenindividuell die Genauigkeit der in Figur 2 exemplarisch dargestellten Blutviskositäts-/Hämatokrit-Kurven verbessert.

Die Bestimmung des Hämatokrits und/oder der Hämoglobinwertes kann mit Mitteln der Dialysemaschine erfolgen, d.h. es sind vorzugsweise keine eigens dafür vorgesehenen Zusatzelemente vorhanden.

Die Aufkonzentration des Blutes, d.h. die Erhöhung des Hämatokrits ΔHKT mit der Zeit erfolgt bei unveränderten Behandlungsparametern während der Dialysebehandlung in erster Näherung konstant.

Steigt der gemessene Hämatokrit unerwartet und abrupt oder in einem fließenden Übergang steiler an, übersteigt also ΔHKT/Δt einen Grenzwert, so ist dies als Erhöhung der Viskosität aufgrund der nicht-newtonschen Effekte an der Kapillaroberfläche interpretierbar. Stellt man während der Behandlung fest, dass der zeitliche Anstieg einen Grenzwert übersteigt, so wird mittels einer Steuer- oder Regelungseinheit der Dialysemaschine die Blutflussrate und/oder die Dilutionsrate, vorzugsweise die Predilutionsrate so geändert, dass sich der erwartete ΔHKT/Δt wieder einstellt oder in einem bestimmten Sollwertbereich liegt, der z.B. als Fenster um einen Sollwert vorliegen kann. Vorzugsweise wird bei einer Grenzwertüberschreitung die Blutflussrate und/oder die Ultrafiltrationsrate verringert und/oder die Dilutionsrate erhöht.

Vorzugsweise findet eine Regelung des ΔHKT/Δt statt, wobei als Stellgrößen die Blutflussrate und/oder die Dilutionsrate und/oder die Ultrafiltrationsrate dienen können.

Zwar ist grundsätzlich eine Änderung der Ultrafiltrationsrate zur Einstellung des ΔHKT/Δt denkbar und von der Erfindung mit umfasst. Bevorzugt ist es jedoch, wenn diese konstant bleibt oder einem vorgegebenen Profil folgt, um am Behandlungsende den gewünschten Gewichtsverlust erreichen zu können.

## Patentansprüche

1. Dialysemaschine mit einem extrakorporalen Kreislauf, in dem sich ein Dialysator befindet, der eine von Blut durchströmte blutseitige Kammer aufweist, sowie ein in Strömungsrichtung des Blutes stromaufwärts der blutseitigen Kammer befindlicher erster Drucksensor, der einen ersten Druckwert ermittelt, und ein in Strömungsrichtung des Blutes stromabwärts der blutseitigen Kammer befindlichen zweiter Drucksensor, der einen zweiten Druckwert ermittelt, **dadurch gekennzeichnet,** **dass** die Dialysemaschine erste Mittel aufweist, die die Druckdifferenz zwischen dem zweiten und dem ersten Druckwert ermitteln, **dass** die Dialysemaschine zweite Mittel aufweist, die die dynamische Viskosität des Blutes basierend auf der ermittelten Druckdifferenz, der Blutflussrate durch die blutseitige Kammer und einer oder mehrerer charakteristischer Eigenschaften des Dialysators ermitteln, **dass** die Dialysemaschine dritte Mittel aufweist, die den Hämatokrit oder den Hämoglobinwert des Blutes im Dialysator basierend auf der ermittelten Viskosität ermitteln und **dass** die Dialysemaschine eine Steuer- oder Regelungseinheit aufweist, die derart ausgebildet ist, dass diese die Blutflussrate und/oder die Dilutionsrate und/oder die Ultrafiltrationsrate derart einstellt, dass die zeitliche Veränderung des Hämatokrits und/oder des Hämoglobinwertes einen Grenzwert nicht übersteigt oder in einem Sollwertbereich liegt.

2. Dialysemaschine nach Anspruch 1, **dadurch gekennzeichnet, dass** die Steuer- oder Regelungseinheit derart ausgebildet ist, dass die Ultrafiltrationsrate nicht zur Einstellung der Veränderung des Hämatokrits und/oder des Hämoglobinwertes herangezogen wird.

3. Dialysemaschine nach Anspruch 1 oder 2, **dadurch gekennzeichnet, dass** der Grenzwert oder der Sollwertbereich fest vorgegeben ist oder experimentell ermittelt ist.

4. Dialysemaschine nach einem der vorhergehenden Ansprüche, **dadurch gekennzeichnet, dass** die Eigenschaften des Dialysators den sich bei laminarer Strömung ergebenden Strömungswiderstand des Dialysators umfassen.

5. Dialysemaschine nach einem der vorhergehenden Ansprüche, **dadurch gekennzeichnet, dass** die blutseitige Kammer des Dialysators aus dem Lumen einer Mehrzahl von Kapillaren besteht und dass die Eigenschaften des Dialysators die Länge der Kapillaren und/oder deren Innenabmessungen und/oder deren Anzahl und/oder deren Form und/oder deren Wandstruktur und/oder die Rauigkeit der Kapillarwand umfassen.

6. Dialysemaschine nach einem der vorhergehenden Ansprüche, **dadurch gekennzeichnet, dass** die dritten Mittel einen Speicher aufweisen, in dem ein Zusammenhang zwischen Hämatokrit oder Hämoglobinwert des Blutes und der Viskosität abgelegt ist und dass die dritten Mittel derart ausgeführt sind, dass der Hämatokrit oder der Hämoglobinwert des Blutes basierend auf diesem Zusammenhang bestimmt wird.

7. Dialysemaschine nach Anspruch 6, **dadurch gekennzeichnet, dass** der Zusammenhang fest vorgegeben ist.

8. Dialysemaschine nach Anspruch 6, **dadurch gekennzeichnet, dass** der Zusammenhang mittels eines Korrekturfaktors patientenindividuell bestimmt ist.

9. Dialysemaschine nach einem der Ansprüche 6 bis 8, **dadurch gekennzeichnet, dass** die dritten Mittel derart ausgebildet sind, dass der Hämatokrit oder Hämoglobinwert durch Interpolation oder durch Extrapolation bestimmt wird.

**10.** Dialysemaschine nach einem der vorhergehenden Ansprüche, **dadurch gekennzeichnet, dass** die Dialysemaschine Mittel zum Einlesen der charakteristischen Eigenschaften des Dialysators aufweist, die mit den zweiten Mitteln zur Bestimmung der Viskosität in Verbindung stehen.

**11.** Dialysemaschine nach einem der vorhergehenden Ansprüche, **dadurch gekennzeichnet, dass** die ersten bis dritten Mittel ohnehin vorhandene Bestandteile einer Dialysemaschine sind, so dass keine zusätzlichen Elemente zur Durchführung der von den ersten bis dritten Mitteln vorgenommenen Maßnahmen erforderlich sind.

**12.** Dialysemaschine nach einem der vorhergehenden Ansprüche, **dadurch gekennzeichnet, dass** die charakteristischen Eigenschaften des Dialysators durch Messung mit Blut oder durch Messung mit einer anderen Flüssigkeit, insbesondere durch Messung mit einer Spülflüssigkeit erhalten sind.

**13.** Dialysemaschine nach einem der vorhergehenden Ansprüche, **dadurch gekennzeichnet, dass** die Dialysemaschine eine von Dialyselösung durchströmte dialysatseitige Kammer sowie einen in Strömungsrichtung der Dialyselösung stromaufwärts der dialysatseitigen Kammer befindlichen dritten Drucksensor zur Ermittlung eines dritten Druckwertes und einen in Strömungsrichtung der Dialyselösung stromabwärts der dialysatseitigen Kammer befindlichen vierten Drucksensor zur Ermittlung eines vierten Druckwertes aufweist.

**14.** Dialysemaschine nach einem der vorhergehenden Ansprüche, **dadurch gekennzeichnet, dass** es sich bei dem Dialysator um einen Hämodialysator oder um einen Hämofilter handelt.

**15.** Dialysemaschine nach einem der vorhergehenden Ansprüche, **dadurch gekennzeichnet, dass** die Dialysemaschine eine Predilutionsleitung aufweist, die in Strömungsrichtung des Dialysators stromaufwärts des Dialysators in den extrakorporalen Kreislauf mündet und dass es sich bei der Dilutionsrate um die Infusionsrate eines durch die Predilutionsleitung zugeführten Substitutionsfluids handelt.

**Claims**

**1.** A dialysis machine having an extracorporeal circuit in which a dialyzer is located which has a chamber on the blood side which is flowed through by blood and a first pressure sensor, which is located upstream of the chamber on the blood side in the direction of flow of the blood and which determines a first pressure value and a second pressure sensor, which is located downstream of the chamber on the blood side in the direction of flow of the blood and which determines a second pressure value, **characterized in that** the dialysis machine has first means which determine the pressure difference between the second pressure value and the first pressure value; the dialysis machine has second means which determine the dynamic viscosity of the blood on the basis of the determined pressure difference, of the blood flow rate through the chamber on the blood side and of one or more characteristic properties of the dialyzer; the dialysis machine has third means which determine the hematocrit or the hemoglobin value of the blood in the dialyzer on the basis of the determined viscosity; and the dialysis machine has a control or regulation unit which is configured such that it sets the blood flow rate and/or the dilution rate and/or the ultrafiltration rate such that the time change of the hematocrit and/or of the hemoglobin value does not exceed a limit value or lies within a desired value range.

**2.** A dialysis machine in accordance with claim 1, **characterized in that** the control or regulation unit is configured such that the ultrafiltration rate is not used to set the change of the hematocrit and/or of the hemoglobin value.

**3.** A dialysis machine in accordance with claim 1 or claim 2, **characterized in that** the limit value or the desired value range is fixedly predefined or is determined experimentally.

**4.** A dialysis machine in accordance with one of the preceding claims, **characterized in that** the properties of the dialyzer comprise the flow resistance of the dialyzer which results with a laminar flow.

**5.** A dialysis machine in accordance with one of the preceding claims, **characterized in that** the dialyzer chamber on the blood side comprises the lumen of a plurality of capillaries; and **in that** the properties of the dialyzer comprise the length of the capillaries and/or their inner dimensions and/or their number and/or their shape and/or their wall structure and/or the roughness of the capillary wall.

**6.** A dialysis machine in accordance with one of the preceding claims, **characterized in that** the third means have a memory in which a relationship between the hematocrit or hemoglobin value of the blood and the viscosity is stored and that the third means are configured such that the hematocrit or the hemoglobin value of the blood is determined on the basis of this relationship.

**7.** A dialysis machine in accordance with claim 6, **characterized in that** the relationship is fixedly preset.

**8.** A dialysis machine in accordance with claim 6, **characterized in that** the relationship is determined by means of a correction factor on an individual patient basis.

**9.** A dialysis machine in accordance with one of the claims 6 to 8, **characterized in that** the third means are configured such that the hematocrit or hemoglobin value is determined by interpolation or by extrapolation.

**10.** A dialysis machine in accordance with one of the preceding claims, **characterized in that** the dialysis machine has means for reading in the characteristic properties of the dialyzer which are in communication with the second means for determining the viscosity.

**11.** A dialysis machine in accordance with one of the preceding claims, **characterized in that** the first to third means are components of a dialysis machine anyway present so that no additional elements are required to carry out the measures carried out by the first to third means.

**12.** A dialysis machine in accordance with one of the preceding claims, **characterized in that** the characteristic properties of the dialyzer are obtained by measurement with blood or by measurement with another fluid, in particular by measurement with a flushing liquid.

**13.** A dialysis machine in accordance with one of the preceding claims, **characterized in that** the dialysis machine has a chamber flowed through by dialysis solution on the dialyzate side and a third pressure sensor, which is located upstream of the chamber at the dialyzate side in the direction of flow of the dialysis solution, for determining a third pressure value and a fourth pressure sensor, located downstream of the chamber at the dialyzate side in the direction of flow of the dialysis solution, for determining a fourth pressure value.

**14.** A dialysis machine in accordance with one of the preceding claims, **characterized in that** the dialyzer is a hemodialyzer or a hemofilter.

**15.** A dialysis machine in accordance with one of the preceding claims, **characterized in that** the dialysis machine has a predilution line which opens into the extracorporeal circuit upstream of the dialyzer in the direction of flow of the dialyzer; and **in that** the dilution rate is the infusion rate of a substitution fluid supplied through the predilution line.

**Revendications**

**1.** Machine de dialyse comprenant un circuit extracorporel, dans lequel se trouve un dialyseur, qui comporte une chambre côté sang traversée par du sang, ainsi qu'un premier capteur de pression situé en amont de la chambre côté sang dans le sens d'écoulement du sang, qui détermine une première valeur de pression, et un deuxième capteur de pression situé en aval de la chambre côté sang dans le sens d'écoulement du sang, qui détermine une deuxième valeur de pression,
**caractérisée**
**en ce que** la machine de dialyse comporte des premiers moyens, qui déterminent la différence de pression entre la deuxième et la première valeur de pression,
**en ce que** la machine de dialyse comporte des deuxièmes moyens, qui déterminent la viscosité dynamique du sang sur la base de la différence de pression déterminée, du débit sanguin à travers la chambre côté sang et d'une ou de plusieurs propriétés caractéristiques du dialyseur,
**en ce que** la machine de dialyse comporte des troisièmes moyens, qui déterminent l'hématocrite ou la valeur d'hémoglobine du sang dans le dialyseur sur la base de la viscosité déterminée et
**en ce que** la machine de dialyse comporte une unité de commande ou de régulation, qui est réalisée de telle manière qu'elle règle le débit sanguin et/ou le débit de dilution et/ou le débit d'ultrafiltration de telle manière que la variation temporelle de l'hématocrite et/ou de la valeur d'hémoglobine ne dépasse pas une valeur limite ou se situe dans une plage de valeurs de consigne.

**2.** Machine de dialyse selon la revendication 1, **caractérisée en ce que** l'unité de commande ou de régulation est réalisée de telle manière que le débit d'ultrafiltration n'est pas pris en compte pour le réglage de la variation de l'hématocrite et/ou de la valeur d'hémoglobine.

**3.** Machine de dialyse selon la revendication 1 ou 2, **caractérisée en ce que** la valeur limite ou la plage de valeurs de consigne est définie de manière fixe ou est déterminée de manière expérimentale.

**4.** Machine de dialyse selon l'une des revendications précédentes, **caractérisée en ce que** les propriétés du dialyseur comprennent la résistance à l'écoulement du dialyseur résultant lors d'un écoulement laminaire.

**5.** Machine de dialyse selon l'une des revendications précédentes, **caractérisée en ce que** la chambre côté sang du dialyseur se compose de la lumière d'une pluralité de capillaires et **en ce que** les propriétés du dialyseur comprennent la longueur des capillaires et/ou leurs dimensions intérieures et/ou leur nombre et/ou leur forme et/ou leur structure de paroi et/ou la rugosité de la paroi capillaire.

**6.** Machine de dialyse selon l'une des revendications précédentes, **caractérisée en ce que** les troisièmes moyens comportent une mémoire, dans laquelle un rapport entre l'hématocrite ou la valeur d'hémoglobine du sang et la viscosité est enregistré et **en ce que** les troisièmes moyens sont réalisés de telle manière que l'hématocrite ou la valeur d'hémoglobine du sang sont déterminés sur la base de ce rapport.

**7.** Machine de dialyse selon la revendication 6, **caractérisée en ce que** le rapport est défini de manière fixe.

**8.** Machine de dialyse selon la revendication 6, **caractérisée en ce que** le rapport est déterminé de manière personnalisée pour le patient au moyen d'un facteur de correction.

**9.** Machine de dialyse selon l'une des revendications 6 à 8, **caractérisée en ce que** les troisièmes moyens sont réalisés de telle manière que l'hématocrite ou la valeur d'hémoglobine est déterminé(e) par interpolation ou par extrapolation.

**10.** Machine de dialyse selon l'une des revendications précédentes, **caractérisée en ce que** la machine de dialyse comporte des moyens destinés à la lecture des propriétés caractéristiques du dialyseur, qui sont en liaison avec les deuxièmes moyens pour déterminer la viscosité.

**11.** Machine de dialyse selon l'une des revendications précédentes, **caractérisée en ce que** les premiers aux troisièmes moyens sont dans tous les cas des éléments constitutifs existants d'une machine de dialyse, de sorte qu'aucun élément supplémentaire n'est requis pour exécuter les mesures exécutées par les premiers aux troisièmes moyens.

**12.** Machine de dialyse selon l'une des revendications précédentes, **caractérisée en ce que** les propriétés caractéristiques du dialyseur sont obtenues par une mesure avec du sang ou par une mesure avec un autre liquide, en particulier par une mesure avec un liquide de rinçage.

**13.** Machine de dialyse selon l'une des revendications précédentes, **caractérisée en ce que** la machine de dialyse comporte une chambre côté dialysat traversée par une solution de dialyse ainsi qu'un troisième capteur de pression situé en amont de la chambre côté dialysat dans le sens d'écoulement de la solution de dialyse pour déterminer une troisième valeur de pression et un quatrième capteur de pression situé en aval de la chambre côté dialysat dans le sens d'écoulement de la solution de dialyse pour déterminer une quatrième valeur de pression.

**14.** Machine de dialyse selon l'une des revendications précédentes, **caractérisée en ce que** le dialyseur est un hémodialyseur ou un hémofiltre.

**15.** Machine de dialyse selon l'une des revendications précédentes, **caractérisée en ce que** la machine de dialyse comporte une conduite de prédilution, qui débouche dans le circuit extracorporel en amont du dialyseur dans le sens d'écoulement du dialyseur et **en ce que** le débit de dilution est le débit de perfusion d'un fluide de substitution alimenté par la conduite de prédilution.

Figur 1

a)

10

b)

10

c)

10

Figur 2